# EUROPÄISCHE PATENTANMELDUNG

(11) **EP 3 760 158 A1**
(43) Veröffentlichungstag der Anmeldung: **06.01.2021**
(21) Anmeldenummer: 20173298.9
(22) Anmeldetag: 19.06.2015
(51) Int. Cl.: A61C 1/08, A61C 9/00, A61C 13/00

(54) **VORRICHTUNG ZUR VERWENDUNG IN EINEM VERFAHREN ZUM HERSTELLEN EINER ZAHNIMPLANTATSTRUKTUR**

(30) Priorität: 19.06.2014 DE 202014004912 U; 09.03.2015 DE 202015001753 U
(62) Teilanmeldung aus: 15172938.1
(71) Anmelder: R+K CAD CAM Technologie GmbH & Co. KG, 12681 Berlin (DE)
(72) Erfinder: Holtzhausen, Stefan, 01705 Freital (DE); Sembdner, Philipp, 01069 Dresden (DE); Ellmann, Daniel, 15370 Fredersdorf (DE); Klar, Andreas, 12683 Berlin (DE)
(74) Vertreter: Eisenführ Speiser

(57) **Zusammenfassung**

Beschrieben wird eine Vorrichtung zur Verwendung in einem Verfahren zum Herstellen einer Zahnimplantatstruktur, mit einer Abtasteinrichtung (20), die vorgesehen ist, den Kiefer eines Patienten abzutasten und daraus entsprechende Bilddaten zu erzeugen, einer ersten Verarbeitungseinrichtung (22), die ausgebildet ist, in den Bilddaten aufgrund einer Festlegung einer Position, einer Orientierung und eines Raumes für die Anordnung eines Implantates Implantatdaten zu erzeugen, die Parameter für die Anordnung des Implantats wie insbesondere die Abmessungen des hierfür festgelegten Raumes angeben, einer zweiten Verarbeitungseinrichtung (26), die ausgebildet ist, die Implantatdaten mit abgespeicherten Daten einer Vielzahl von unterschiedlichen Implantatausführungen zu vergleichen und als Ergebnis dieses Vergleiches mindestens eine passende Implantatausführung aus der Vielzahl der unterschiedlichen Implantatausführungen anzugeben, und einer Transformationseinrichtung (34), die ausgebildet ist, die Implantatdaten auf der Grundlage eines Referenzkoordinatensystems in Maschinendaten für eine im Zusammenhang mit dem Einsetzen des Implantates in den Kiefer zu verwendende Bearbeitungsmaschine (38) zu transformieren. Ferner wird ein Verfahren zur Herstellung einer Zahnimplantatstruktur insbesondere unter Verwendung der Vorrichtung beschrieben.

## Beschreibung

Die Erfindung betrifft eine Vorrichtung zur Verwendung in einem Verfahren zum Herstellen einer Zahnimplantatstruktur sowie ein Verfahren zur Herstellung einer Zahnimplantatstruktur.

Bei der durchgängig rechnergestützten Planung einer dentalen Versorgung, die anhand von individuellen 3D-Patientenvolumendaten erfolgt, besteht die Problemstellung in der kontinuierlichen Informationsversorgung und -transformation von der Planung am Patientendatensatz bis hin zur Fertigung. Bisher praktizierte Abläufe leiden an einem informationellen Bruch in der digitalen Prozesskette. Die Planungsdaten (z.B. Position und Orientierung von Implantaten und Bohrhülsen) liegen zunächst im CT-Koordinatensystem vor und können somit nicht direkt für die Fertigung verwendet werden. Sie müssen in ein einheitliches und bekanntes Koordinatensystem überführt werden, um einen zweckmäßigen durchgängig digitalen Workflow zu erzielen. Notwendige Referenzobjekte für diese Transformation müssen dabei sowohl im 3D-Volumendatensatz, als auch im Datensatz der Positionierschablone bekannt sein.

Patientenindividuelle Bohrhilfen zur passgenauen Implantation von Zahnimplantaten gelten als Stand der Technik (EP 1 101 451 A2). Zur Herstellung individueller Positionierschablonen für die Dentalimplantation erfolgt die manuelle Anfertigung einer Scanschablone mit sog. Scanzähnen über einen analogen oder digitalen Zahnabdruck. In diese Schablone wird vom Zahntechniker ein Referenzkörper (Referenzplatte, Referenzpins etc.) eingearbeitet. Nach der Digitalisierung des Patientenunterkiefers und/oder -oberkiefers mit aufgesetzter Scanschablone mittels Oberflächenscan und/oder bildgebender Verfahren (CT, DVT) erfolgt eine rechnergestützte Implantatplanung (EP 2 072 018 A1). Über den Referenzkörper in Form einer Platte erfolgt eine interaktive Ausrichtung der Implantate zum Koordinatensystem der Positionierschablone. Zum gegenwärtigen Zeitpunkt gibt es im weiteren Verlauf der Prozesskette zwei prinzipielle Vorgehensweisen. Zum einen kann der Datensatz direkt online an den entsprechenden Systemanbieter versendet werden. Nach 5 bis 10 Tagen erhält der Zahntechniker die zentral gefertigte Positionierschablone. Es wird somit zusätzlich zur Scanschablone die eigentliche Positionierschablone angefertigt. Häufig lässt die Passfähigkeit zu wünschen übrig, und die Kosten für die Bereitstellung sind enorm. Zum anderen können Implantatposition und -ausrichtung als Zahlenwerte auf Papier ausgedruckt werden. Diese Werte werden dann an einem separaten analogen Bohrtisch eingestellt und die Bohrungen vorgenommen (DE 196 29 708 A1). Hierbei kann eine neue Positionierschablone angefertigt werden oder aber die Scanschablone in eine Positionierschablone umgearbeitet werden.

Andere Lösungen präferieren die Überführung der Ergebnisse der Planung in ein Datenfile, dessen Informationen auf Papier ausgedruckt werden. Die ermittelten Werte der Planung werden danach an einem 3D-Tisch wiederum händisch eingegeben. Durch den Bruch in der Informationskette ist mit einem hohen zeitlichen Aufwand zu rechnen; der Ablauf ist zudem fehleranfällig. Die Kosten für die Investition des 3D-Tischs sind enorm; dieser ist allerdings für die Positionierschablonenfertigung unbedingt notwendig.

Bei einer durchgängig rechnergestützten Arbeitsweise werden gegenwärtig mehrere unterschiedliche Softwarelösungen (SKYplanX, Rhino, Delcam) nacheinander bis zur CNC-Herstellung der Positionierschablone genutzt. Dieser Prozess ist einem Zahntechniker sehr schwer vermittelbar, birgt enorme Fehlerquellen und setzt die Investition mehrerer preisintensiver unterschiedlicher Softwarelösungen voraus.

Aufgabe der vorliegenden Erfindung ist es, die zuvor aufgezeigten Nachteile bei der Herstellung einer Zahnimplantatstruktur zu überwinden und dadurch insbesondere die Herstellung von Zahnimplantatstrukturen zu vereinfachen.

Gelöst wird die Aufgabe gemäß einem ersten Aspekt der Erfindung mit einer Vorrichtung zur Verwendung in einem Verfahren zum Herstellen einer Zahnimplantatstruktur, mit einer Abtasteinrichtung, die vorgesehen ist, den Kiefer eines Patienten abzutasten und daraus entsprechende Bilddaten zu erzeugen, einer ersten Verarbeitungseinreichung, die ausgebildet ist, in den Bilddaten aufgrund einer Festlegung einer Position, einer Orientierung und eines Raumes für die Anordnung eines Implantates Implantatdaten zu erzeugen, die Parameter für die Anordnung des Implantats wie insbesondere die Abmessungen des hierfür festgelegten Raumes angeben, einer zweiten Verarbeitungseinrichtung, die ausgebildet ist, die Implantatdaten mit abgespeicherten Daten einer Vielzahl von unterschiedlichen Implantatausführungen zu vergleichen und als Ergebnis dieses Vergleiches mindestens eine passende Implantatausführung aus der Vielzahl der unterschiedlichen Implantatausführungen anzugeben, und einer Transformationseinrichtung, die ausgebildet ist, die Implantatdaten auf der Grundlage eines Referenzkoordinatensystems in Maschinendaten für eine im Zusammenhang mit dem Einsetzen des Implantates in den Kiefer zu verwendende Bearbeitungsmaschine zu transformieren.

Bevorzugt ist die erste Verarbeitungseinrichtung ausgebildet, die Implantatdaten unter Berücksichtigung eines vorbestimmten Mindestabstandes zu einem benachbarten Hindernis wie insbesondere einem benachbarten Zahn, Implantat und/oder Nerv zu erzeugen, sodass sie für die Anordnung eines Implantats nur einen festgelegten Raum definieren, der in einem Abstand zu einem benachbarten Hindernis liegt, welcher mindestens dem vorbestimmten Mindestabstand entspricht. Des Weiteren ist bevorzugt die erste Verarbeitungseinrichtung ausgebildet, die Implantatdaten unter Berücksichtigung eines vorbestimmten Mindestabstandes zu den Außenseiten des Kiefers zu erzeugen, sodass sie für die Aufnahme eines Implantats nur einen solchen Raum definieren, der in einem Abstand zu den Außenseiten des Kiefers liegt, welcher mindestens dem vorbestimmten Mindestabstand entspricht.

Bevorzugt kann eine dritte Verarbeitungseinrichtung vorgesehen sein, die ausgebildet ist, die Implantatdaten unter Verwendung der Daten der von der zweiten Verarbeitungseinrichtung angegebenen passenden Implantatausführung entsprechend anzupassen.

Ferner kann bevorzugt die zweite Verarbeitungseinrichtung ausgebildet sein, als Ergebnis des Vergleiches aus der Vielzahl der unterschiedlichen Implantatausführungen eine Gruppe von passenden Implantatausführungen zur Auswahl durch den Anwender anzugeben.

Zweckmäßigerweise wird eine Anzeige zur Darstellung eines Bildes des Kiefers des Patienten aufgrund der Bilddaten verwendet.

Des Weiteren sollte bevorzugt eine Datenbank zur Abspeicherung der Daten einer Vielzahl von unterschiedlichen Implantatausführungen vorgesehen werden.

Gemäß einer weiteren bevorzugten Ausführung weist die Vorrichtung einen Referenzabschnitt, in dem Referenzelemente zur Definition eines Referenzkoordinatensystems anzuordnen sind, einen Schablonenabschnitt, der zur lösbaren Befestigung am Kiefer eines Patienten und zur Aufnahme oder Ausbildung mindestens eines Zahnersatzteiles vorgesehen ist, und Haltemittel auf, die in einer definierten Position in Bezug auf die Referenzelemente angeordnet und für ein lösbares Einspannen in einer Bearbeitungsmaschine, insbesondere einer CNC-Fräsmaschine, zum Einarbeiten mindestens eines Funktionselementes, bevorzugt einer Bohrung, zumindest in den Schablonenabschnitt ausgebildet ist. Bei dieser Ausführung gemäß Anspruch 5 handelt es sich im Übrigen auch um einen weiteren selbstständigen Erfindungsgedanken. Bevorzugt sind die Haltemittel am oder im Referenzabschnitt vorgesehen und/oder weisen Aussparungen und/oder Löcher auf. Für eine eindeutige Zuordnung sollte bevorzugt für die Referenzelemente eine asymmetrische Anordnung vorgesehen sein. Des Weiteren sollten bevorzugt im Referenzabschnitt im Querschnitt kreisförmige Löcher oder Aussparungen zur, bevorzugt lösbar, arretierenden Aufnahme von als Kugeln ausgebildeten Referenzelementen vorgesehen sein.

Zweckmäßigerweise sollte der Referenzabschnitt im Wesentlichen als Platte ausgebildet sein. Bevorzugt können der Referenzabschnitt, der Schablonenabschnitt und die Haltemittel miteinander einstückig ausgebildet sein, alternativ ist es aber auch denkbar, den Referenzabschnitt lösbar am Schablonenabschnitt anzuordnen. Zur Befestigung mindestens eines Zahnersatzteils am Schablonenabschnitt sollten bevorzugt Befestigungsmittel, insbesondere Klebemittel verwendet werden.

Für eine eindeutige Differenzierung bei der Darstellung sollten bevorzugt der Referenzabschnitt, der Schablonenabschnitt und die Haltemittel im Wesentlichen nicht abbildbares Material und dagegen die Referenzelemente und das mindestens eine Zahnersatzteil im Wesentlichen abbildbares Material aufweisen, sodass bei einer Abbildung auf einem Bildschirm im Wesentlichen nur die Referenzelemente und das mindestens eine Zahnersatzteil erkennbar sind, auf welches es ja im Wesentlichen auch nur ankommt. Im Hinblick auf die Anatomie des Kiefers ist es von Vorteil, den Schablonenabschnitt im Wesentlichen mit einer entsprechend dem Kiefer gekrümmten Formgebung zu versehen. Für eine exakte Ausbildung von Bohrungen im Kiefer sollte bevorzugt der Schablonenabschnitt zur Einarbeitung mindestens eines als Durchgangsbohrung ausgebildeten Funktionselementes für die Aufnahme einer Bohrerführungshülse vorgesehen sein, die eine genaue Führung einer passenden Bohrspitze gestattet. Zur lösbaren Befestigung in einer definierten Anordnung am Kiefer des Patienten sollte bevorzugt der Schablonenabschnitt Befestigungsmittel, insbesondere Ankerpins, aufweisen.

Ferner wird die zuvor angegebene Aufgabe gemäß einem zweiten Aspekt der Erfindung gelöst mit einem Verfahren zur Herstellung einer Zahnimplantatstruktur, insbesondere unter Verwendung einer Vorrichtung gemäß dem ersten Aspekt der Erfindung, mit den Schritten,
A) mit einer Abtasteinrichtung Bilddaten vom Kiefer eines Patienten zu erzeugen,
B) in den Bilddaten aufgrund einer Festlegung der Position, der Orientierung und des Raumes für die Aufnahme eines Implantates Implantatdaten zu erzeugen, die Parameter für die Anordnung des Implantats wie insbesondere die Abmessungen des hierfür festgelegten Raumes angeben,
C) die Implantatdaten mit abgespeicherten Daten einer Vielzahl von unterschiedlichen Implantatausführungen zu vergleichen und als Ergebnis dieses Vergleiches mindestens eine passende Implantatausführung aus der Vielzahl der unterschiedlichen Implantatausführungen anzugeben, und
D) die Implantatdaten auf der Gundlage eines Referenzkoordinatensystems in Maschinendaten für eine im Zusammenhang mit dem Einsetzen des Implantates in den Kiefer zu verwendende Bearbeitungsmaschine zu transformieren.

Vorzugsweise wird ein vorbestimmter Mindestabstand zu einem benachbarten Hindernis wie insbesondere einem benachbarten Zahn, Implantat und/oder Nerv festgelegt und werden im Schritt B die Implantatdaten unter Berücksichtigung dieses vorbestimmten Mindestabstandes erzeugt, sodass sie für die Anordnung eines Implantats nur einen solchen Raum definieren, der in einem Abstand zu einem benachbarten Hindernis liegt, welcher mindestens dem vorbestimmten Mindestabstand entspricht. Des Weiteren sollte bevorzugt ein vorbestimmter Mindestabstand zu den Außenseiten des Kiefers definiert und im Schritt B die Implantatdaten unter Berücksichtigung dieses vorbestimmten Mindestabstandes erzeugt werden, sodass sie für die Aufnahme eines Implantats nur einen solchen Raum definieren, der in einem Abstand zu den Außenseiten des Kiefers liegt, welcher mindestens dem vorbestimmten Mindestabstand entspricht.

Alternativ kann im Schritt C als Ergebnis des Vergleiches aus der Vielzahl der unterschiedlichen Implantatausführungen eine Gruppe von passenden Implantatausführungen zur Auswahl durch den Anwender angegeben werden. Zweckmäßigerweise sollten zwischen dem Schritt C und dem Schritt D die Implantatdaten unter Berücksichtigung der Daten der passenden Implantatausführung entsprechend angepasst werden.

Die zuvor angegebene Aufgabe wird gemäß einem dritten Aspekt der Erfindung gelöst mit einem Verfahren zur Herstellung einer Zahnimplantatstruktur unter Verwendung einer Vorrichtung nach mindestens einem der Ansprüche 5 bis 16, mit den Schritten,
den Schablonenabschnitt mit mindestens einem Zahnersatzteil zu versehen,
von einem Kiefer des Patienten mit dem daran befestigten Schablonenabschnitt Bilddaten zu erzeugen,
auf der Grundlage der in den Bilddaten enthaltenen Daten für die Referenzelemente ein Referenzkoordinatensystem zu definieren,
in den Bilddaten aufgrund einer Festlegung einer Position, einer Orientierung und eines Raumes für die Anordnung eines Implantates entsprechende Implantatdaten zu erzeugen,
die Implantatdaten auf der Grundlage des Referenzkoordinatensystems in Maschinendaten zu transformieren,
die Anordnung aus Schablonenabschnitt und Referenzabschnitt mit den Haltemitteln in eine Bearbeitungsmaschine einzuspannen und
mit Hilfe der Bearbeitungsmaschine unter Verwendung der Maschinendaten mindestens ein Funktionselement, bevorzugt eine Bohrung, zumindest in den Schablonenabschnitt der Vorrichtung einzuarbeiten.

Sofern die Verfahren gemäß zweitem Aspekt und gemäß drittem Aspekt der Erfindung in Kombination verwendet werden, ist es von Vorteil,
vor dem Schritt A den Schablonenabschnitt mit mindestens einem Zahnersatzteil zu versehen,
den Schablonenabschnitt am Kiefer eines Patienten zu befestigen,
zwischen dem Schritt A und dem Schritt B auf der Grundlage der in den Bilddaten enthaltenen Daten für die Referenzelemente ein Referenzkoordinatensystem zu definieren und
nach dem Schritt D die Anordnung aus Schablonenabschnitt und Referenzabschnitt mit den Haltemitteln in eine Bearbeitungsmaschine einzuspannen und mithilfe der Bearbeitungsmaschine unter Verwendung der Maschinendaten mindestens ein Funktionselement, bevorzugt eine Bohrung, zumindest in den Schablonenabschnitt der Vorrichtung einzuarbeiten.

Ferner ist bevorzugt ein weiterer Schritt vorzusehen, den Referenzabschnitt vom Schablonenabschnitt zu trennen.

Schließlich können die zuvor beschriebenen Verfahren bevorzugt in einem Computerprogramm implementiert werden.

Im Übrigen wird hinsichtlich der bevorzugten Ausführungen und Weiterbildungen der Erfindung auf die abhängigen Ansprüche verwiesen.

Die Erfindung zielt auf einen sicheren durchgängig digitalen Daten- und Informationsfluss von der Therapieplanung anhand von CT- und DVT-Schichtbildern bis zur CNC-gestützten Herstellung von Positionierschablonen für Operationen im Dentalbereich (z.B. Implantationen) ab. Die Erfindung ermöglicht eine automatische Transformation der therapierelevanten Planungskoordinaten (z.B. Implantat- und Bohrhülsenpositionen) in ein Fertigungskoordinatensystem insbesondere unter Nutzung von speziellen Referenzobjekten wie beispielsweise Referenzelemente in einem Referenzabschnitt, an dem ein Schablonenabschnitt angeordnet ist.

Gemäß einem bevorzugten Aspekt, der alternativ auch einen eigenständigen Erfindungsgedanken darstellt, bildet Grundlage für die Lösung der zuvor angegebenen Aufgabe ein Referenzabschnitt mit eindeutig definiertem Plattenkoordinatensystem, der während der Herstellung an einem Schablonenabschnitt unlösbar und fest in diese integriert wird. Der Vorteil dabei ist, dass der Referenzabschnitt bei der Befestigung gegenüber dem Schablonenabschnitt relativ frei positionierbar ist. Somit kann der Referenzabschnitt abweichend von der Zahnebene und unter Berücksichtigung der Gegebenheiten im Mundraum angebracht werden. Eine Beeinflussung des Transformationsergebnisses z.B. durch Artefakte in der volumentomografischen Aufnahme kann dadurch minimiert oder eliminiert werden. Der Referenzabschnitt wird bevorzugt aus einem nicht röntgenopaken festen Werkstoff gefertigt und dient somit gleichzeitig zur Stabilisierung des Schablonenabschnittes. Der Referenzabschnitt weist bevorzugt ein eindeutiges, unsymmetrisches Schema zur Aufnahme von (mindestens 3) Referenzelementen aus röntgenopakem Material (Keramik, Si3N4) auf. Die Informationen zu Anzahl, Durchmesser und Lage der Referenzelemente gegenüber dem Plattenkoordinatensystem sind bekannt und werden in einem Datenfile gespeichert.

Nachfolgend wird ein bevorzugtes Ausführungsbeispiel der Erfindung anhand der beiliegenden Zeichnungen näher erläutert. Es zeigen:
- Fig. 1: schematisch im Blockschaltbild eine Vorrichtung für eine durchgängig rechnergestützte Planung einer dentalen Versorgung gemäß einem bevorzugten Ausführungsbeispiel;
- Fig. 2: ein Ablaufdiagramm zur schematischen Darstellung der rechnergestützten Planung einer dentalen Versorgung mithilfe der in Fig. 1 dargestellten Vorrichtung;
- Fig. 3: eine Scanschablone mit daran befestigter Referenzplatte gemäß einem bevorzugten Ausführungsbeispiel der Erfindung in Anordnung auf dem Modell eines Unterkiefers (Fig. 3a) und in alleiniger Darstellung (Fig. 3b);
- Fig. 4: beispielhaft die Abbildung eines Kiefers mit einer Umrissplanung für ein neues Implantat sowie mit Darstellung eines Implantates;
- Fig. 5: die schematische Darstellung eines Bildstapels mit Plattenkoordinaten-system, das durch die Referenzplatte mithilfe der dort angeordneten Referenzkugeln definiert wird; und
- Fig.6: schematisch eine Draufsicht auf die als Positionierschablone zu verwendende Scanschablone mit dort eingebrachten Bohrhülsen.

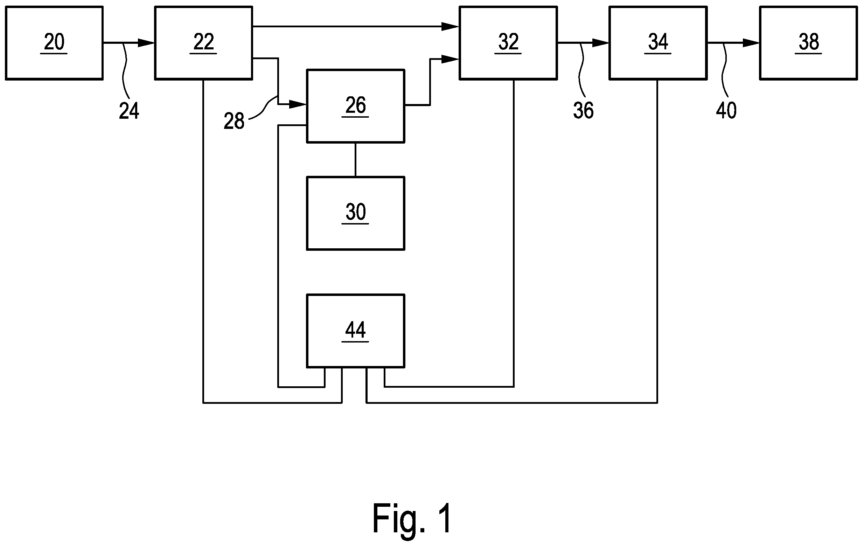

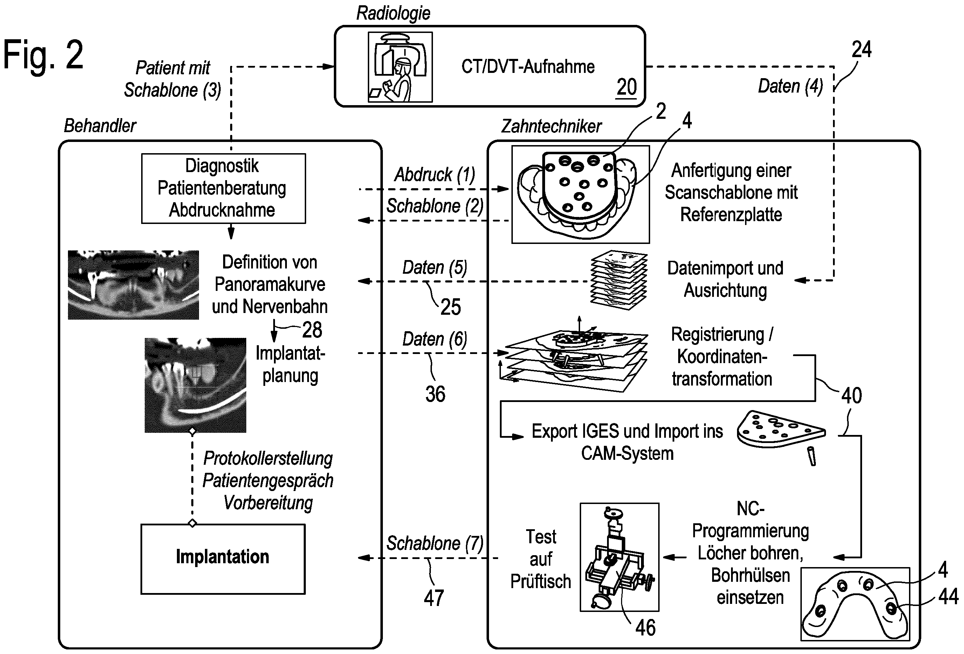

Die Vorrichtung und das Verfahren gemäß dem in den Figuren dargestellten Ausführungsbeispiel zielen auf einen sicheren durchgängig digitalen Daten- und Informationsfluss von der Therapieplanung anhand von CT- und DVT-Schichtbildern bis zur CNC-gestützten Herstellung von Positionierschablonen für Operationen im Dentalbereich (z.B. Implantationen) ab und ermöglichen eine automatische Transformation der therapierelevanten Planungskoordinaten (z.B. Implantat- und Bohrhülsenpositionen) in ein Fertigungskoordinatensystem unter Nutzung von speziellen Referenzobjekten (Referenzkugeln, Referenzplatte, Aufnahmevorrichtung), wie in einer Gesamtschau die Fig. 1, die beispielhaft schematisch eine Vorrichtung zeigt, und die Fig. 2, die beispielhaft ein Ablaufdiagramm zeigt, erkennen lassen.

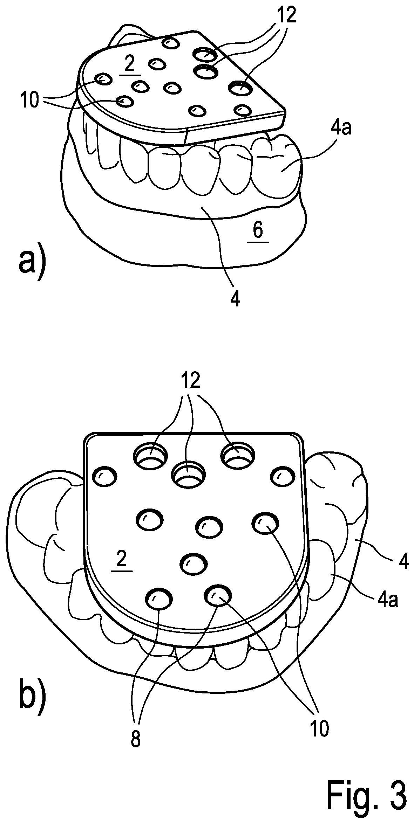

Grundlage hierfür bildet im dargestellten Ausführungsbeispiel eine Referenzplatte 2 mit eindeutig definiertem Plattenkoordinatensystem dar, die während der Herstellung einer Scanschablone 4 unlösbar und fest in diese integriert wird. Für die Anfertigung der Scanschabole 4 wird zunächst entweder von dem betreffenden Kiefer ein Abdruck erzeugt oder der betreffende Kiefer visuell abgetastet. Gemäß dem in den Figuren 2 und 3 dargestellten Ausführungsbeispiel ist die Scanschablone 4 für einen insgesamt zahnlosen Kiefer vorgesehen und somit über die gesamte Länge, die etwa auch der Länge des Kiefers entspricht, mit einer Anordnung von nachgebildeten Zahnersatzteilen 4a versehen. Im Hinblick auf die gekrümmte Form des Kiefers weist auch die Scanschablone 4 im dargestellten Ausführungsbeispiel im Wesentlichen eine entsprechend gekrümmte Formgebung auf. In einem solchen Fall ist es erforderlich, den gesamten Kiefer mit Implantaten zu versehen. Für den alternativen Fall, dass in dem Kiefer an einer bestimmten Stelle nur ein einziges Implantat oder nur einige wenige Implantate vorzusehen sind, ist die Scanschablone nur an dieser Stelle mit entsprechend nachgebildeten Zahnersatzteilen zu versehen.

Fig. 3 zeigt beispielhaft die Scanschablone 4 mit der daran befestigten Referenzplatte 2 in Anordnung auf dem Modell 6 eines Unterkiefers (Fig. 3a) sowie in alleiniger Darstellung (Fig. 3b). Der Vorteil dabei ist, dass die Referenzplatte 2 bei der Befestigung gegenüber der Scanschablone 4 relativ frei positionierbar ist. Somit kann die Referenzplatte 2 abweichend von der Zahnebene und unter Berücksichtigung der Gegebenheiten im Mundraum angebracht werden. Eine Beeinflussung des Transformationsergebnisses z.B. durch Artefakte in der volumentomografischen Aufnahme kann dadurch minimiert oder gar eliminiert werden. Die Referenzplatte 2 wird aus einem nicht röntgenopaken festen Werkstoff gefertigt und dient somit gleichzeitig zur Stabilisierung der Scanschablone 4. Die Referenzplatte weist ein eindeutiges, unsymmetrisches Schema von Bohrungen 8 zur Aufnahme von (mindestens 3) Referenzkugeln 10 aus röntgenopakem Material (Keramik, Si3N4) auf. Die Informationen zu Anzahl, Durchmesser und Lage der Referenzkugeln 10 gegenüber dem Plattenkoordinatensystem sind bekannt und werden in einem Datenfile gespeichert.

Die Referenzplatte 2 weist weiterhin eine asymmetrische Anordnung von (mindestens drei) Montagebohrungen 12 auf, welche eine formschlüssige, wiederholbare Einspannung in einer speziellen dafür angepassten in den Figuren nicht dargestellten Aufnahmevorrichtung ermöglicht. Ein versehentliches Verdrehen der Referenzplatte 2 beim Einlegen ist ausgeschlossen. Die Aufnahmevorrichtung lässt sich einmalig durch Einmessen anhand bekannter technologischer Basen (z.B. Ebenen) in Bezug zur Plattenaufnahme innerhalb einer Werkzeugmaschine positionieren (Fertigungskoordinatensystem).

Die Scanschablone 4 wird, wie bereits zuvor erwähnt, anhand eines Kieferabdrucks, dessen Modell in Fig. 3a beispielhaft dargestellt und mit dem Bezugszeichen "6" bezeichnet ist, und/oder eines digitalen Mundscans angefertigt. Nach Einarbeitung der Referenzplatte 2 mit den Referenzkugeln 10 in die Scanschablone 4 findet eine volumentomografische Aufnahme des betreffenden Kiefers des Patienten mit der Scanschablone 4 statt. Hierzu wird eine Abtasteinrichtung 20 verwendet, wie sie in den Figuren 1 und 2 schematisch angedeutet ist. Die Abtasteinrichtung 20 muss in der Lage sein, den Kiefer zu durchleuchten, und ist deshalb bevorzugt zur Erzeugung von CT-Aufnahmen ausgebildet. Somit werden mit Hilfe der Aufnahmeeinrichtung 20 bevorzugt sog. Schichtaufnahmen generiert, die in bestimmten Abständen voneinander jeweils eine Art Schnittbild darstellen und zusammengesetzt eine perspektivische Abbildung ergeben. Die von der Abtasteinrichtung 20 erzeugten Bilddaten werden in einer ersten Verarbeitungseinrichtung 22 verarbeitet, wobei die Übertragung der Bilddaten in den Figuren 1 und 2 schematisch durch einen Pfeil mit dem Bezugszeichen "24" angedeutet ist. Neben dem Import der Bilddatenbesteht eine Funktion der ersten Verarbeitungseinrichtung 22 darin, die Bilder in verschiedene Perspektiven zu verdrehen und/oder in ihrer Größe zu reduzieren und/oder die Bildanzahl eines Bildstapels zu verringern. Dabei wird in der ersten Verarbeitungseinrichtung 22 auf der Grundlage der in den Bilddaten enthaltenen Daten für die Referenzkugeln 10 ein Referenzkoordinatensystem definiert.

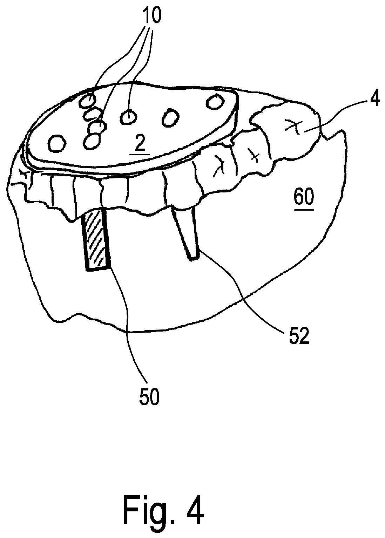

Eine weitere Aufgabe der ersten Verarbeitungseinrichtung 22 besteht darin, in den Bilddaten aufgrund einer Festlegung der Position, der Orientierung und des Raumes für die Aufnahme eines Implantates Implantatdaten zu erzeugen, die Parameter für die Anordnung des späteren Implantats wie insbesondere die Abmessungen des hierfür festgelegten Raumes angeben. Insbesondere bei Festlegung eines vorbestimmten Mindestabstandes zu einem benachbarten Hindernis wie insbesondere einem benachbarten Zahn, Implantat und/oder Nerv werden Implantatdaten unter Berücksichtigung dieses vorbestimmten Mindestabstandes erzeugt, sodass die Implantatdaten für die Anordnung eines Implantats nur einen solchen Raum definieren, der in einem Abstand von dem benachbarten Hindernis liegt, welcher mindestens dem vorbestimmten Mindestabstand entspricht. Des Weiteren sollte ein vorbestimmter Mindestabstand zu den Außenseiten des Kiefers definiert werden, sodass mithilfe der ersten Verarbeitungseinrichtung 22 die Implantatdaten auch unter Berücksichtigung dieses vorbestimmten Mindestabstandes erzeugt werden, indem für die Aufnahme eines Implantats nur ein solcher Raum zur Umrissplanung definiert wird, der in einem Abstand zu den Außenseiten des Kiefers liegt, welcher mindestens dem vorbestimmten Mindestabstand entspricht. Beispielhaft zeigt hierzu Fig. 4 eine aus den zuvor erwähnten Schichtbildern bzw. Bilddaten generierte dreidimensionale Abbildung auf einem Monitor, welche einen Kiefer 60 und eine darauf aufgesetzte Scanschablone 4 mit Referenzplatte 2 zeigt, wobei insbesondere auch deutlich die Referenzkugeln 10 zu erkennen sind. Des Weiteren ist in Fig. 4 mit dem Bezugszeichen "50" schematisch der zuvor bereits erwähnte festzulegende Raum erkennbar dargestellt, innerhalb dessen das spätere Implantat anzuordnen ist. Zur besseren Erkennbarkeit ist der Raum 56 schraffiert dargestellt. Des Weiteren lässt die Abbildung von Fig. 4 beispielhaft noch ein Implantat 52 erkennen, bei dem es sich beispielsweise um ein bereits vorhandenes Implantat handeln kann. In Fig. 2 ist diese interne Weiterverarbeitung der Implantatdaten schematisch durch den Pfeil 25 angedeutet.

Anschließend werden die so von der ersten Verarbeitungseinrichtung 22 verarbeiteten und erzeugten Implantatdaten in einer zweiten Verarbeitungseinrichtung 26 weiterverarbeitet, wobei die Übertragung der Implantatdaten in den Figuren 1 und 2 schematisch durch den Pfeil 28 angedeutet ist. Die Weiterverarbeitung der Implantatdaten in der zweiten Verarbeitungseinrichtung 26 besteht darin, die Implantatdaten mit in einer Datenbank 30 abgespeicherten Daten für eine Vielzahl von unterschiedlichen Implantatausführungen zu vergleichen und als Ergebnis dieses Vergleiches mindestens eine passende Implantatausführung oder auch eine Gruppe von passenden Implantatausführungen zur Auswahl durch den Anwender aus der Vielzahl der unterschiedlichen Implantatausführungen anzugeben.

In einer dritten Verarbeitungseinrichtung 32 (Fig. 1) werden die von der ersten Verarbeitungseinrichtung 22 ausgegebenen Implantatdaten unter Verwendung der Daten der von der zweiten Verarbeitungseinrichtung 26 angegebenen passenden Implantatausführung(en) entsprechend angepasst. Die ausgewählte passende Implantatausführung wird nun visualisiert und virtuell in dem besagten Raum 50 angeordnet, indem in die Bilddaten die Daten der ausgewählten passenden Implantatausführung entsprechend eingearbeitet werden. Dementsprechend kann es sich bei dem in Fig. 4 abgebildeten Implantat 52 alternativ auch um ein derartiges visuelles Implantat handeln, das dann als Vorlage für das spätere Einsetzen eines realen Implantates dient.

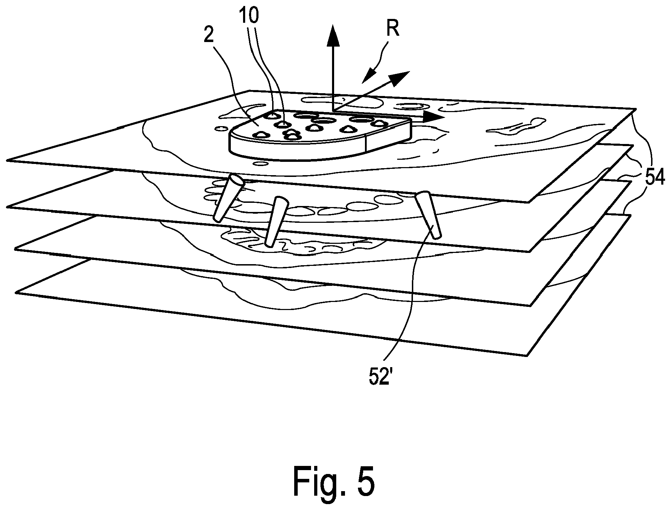

In Fig. 5 ist zur Veranschaulichung ein Bildstapel 54 dargestellt, der beispielhaft aus vier Schichtbildern besteht. Wie Fig. 5 des weiteren schematisch erkennen lässt, wird für die Bilddaten und die Implantatkoordinaten ein Referenzplattenkoordinatensystem verwendet, das durch die Referenzplatte 2 definiert ist. Die mit dem Bezugszeichen "52"' in Fig. 5 gekennzeichneten Elemente bilden geplante visualisierte Implantate, die in einer gewünschten Ausrichtung zur Referenzplatte 2 und somit auch in Bezug auf das Referenzplattenkoordinatensystem R orientiert sind.

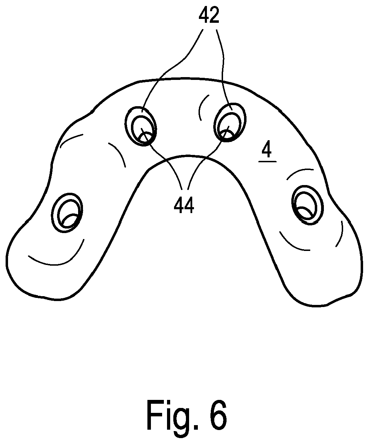

Die so von der dritten Verarbeitungseinrichtung 32 angepassten Implantatdaten werden dann in einer Transformationseinrichtung 34 auf der Grundlage des Referenzkoordinatensystems in Maschinendaten für eine im Zusammenhang mit dem Einsetzen des Implantates in den Kiefer zu verwendende Bearbeitungsmaschine 38 transformiert, die bevorzugt als CNC-Maschine ausgebildet ist. Hierzu wird die Anordnung aus Referenzplatte 2 und Scanschablone 4 mithilfe der Montagebohrungen 12 (Fig. 3) in die Bearbeitungsmaschine 38 eingespannt und mit deren Hilfe unter Verwendung der Maschinendaten von der Transformationseinrichtung 34 Funktionselemente, die im hier beschriebenen Ausführungsbeispiel gemäß Figur 6 als Bohrung 42 ausgebildet sind, in die Scanschablone 4 eingearbeitet. In die in der Scanschablone 4 eingearbeiteten Bohrungen 42 wird jeweils eine ebenfalls in Figur 6 schematisch dargestellte Bohrhülse 44 eingesetzt, die zur Führung einer in den Figuren nicht gezeigten Bohrspitze dient. Die Übertragung der Daten zur Transformationseinrichtung 34 ist in Figuren 1 und 2 schematisch durch den Pfeil 36 und von der Transformationseinrichtung 34 zur Bearbeitungsmaschine 38 schematisch durch den Pfeil 40 angedeutet.

Nach einem Test auf einem Prüftisch 46 wie in Fig. 2 schematisch angedeutet ist, wird die Referenzplatte 2 von der Scanschablone 4 getrennt.

Anschließend wird in den Kiefer des Patienten an der gewünschten Stelle eine Bohrung eingebracht, in der dann später das Implantat angeordnet wird. Hierzu wird die Scanschablone 4 auf den betreffenden Kiefer aufgesetzt und übernimmt nun die Aufgabe einer Positionierschablone, indem die in die Bohrung 42 in der Scanschablone 4 eingesetzte Bohrhülse 44 (Figur 6) zur exakten Führung einer passenden Bohrspitze eines ebenfalls in den Figuren nicht dargestellten Bohrwerkzeuges dient, wodurch die Einbringung der Bohrung exakt in der gewünschten Ausrichtung und an der gewünschten Stelle im Kiefer gewährleistet wird. Dieser im vorliegend beschriebenen Ausführungsbeispiel letzte Verfahrensschritt ist in Fig. 2 schematisch durch den Pfeil 47 angedeutet.

Um zumindest die zuvor beschriebenen relevanten Datenverarbeitungsschritte visuell zu überwachen und auch beeinflussen zu können, ist ein Terminal 44 mit Monitor und Tastatur vorgesehen, das insbesondere an die Verarbeitungseinrichtungen 22, 26 und 32 und die Transformationseinrichtung 34 angeschlossen ist, wobei sich mit dem Monitor u.a.die in Figur 4 schematisch gezeigte Abbildung darstellen lässt.

Ferner sei an dieser Stelle noch ergänzend angemerkt, dass insbesondere die Verarbeitungseinrichtungen 22, 26, 32, die Datenbank 30, die Transformationseinrichtung 34 und das Eingabeterminal 44 zumindest teilweise oder auch vollständig Bestandteile einer elektronischen Datenverarbeitungsanlage sein können.

Das zuvor beschriebene Verfahren zielt auf einen sicheren durchgängig digitalen Daten- und Informationsfluss von der Therapieplanung anhand von CT- und DVT-Schichtbildern bis zur CNC-gestützten Herstellung von Positionierschablonen für Operationen im Dentalbereich (z.B. Implantationen) ab und ermöglicht eine automatische Transformation des therapierelevanten Planungskoordinatensystems (z.B. Implantat- und Bohrhülsenpositionen) in ein Fertigungskoordinatensystem unter Nutzung von speziellen Referenzobjekten (Referenzkugeln 10, Referenzplatte 2, Aufnahmevorrichtung).

Eine Grundlage stellt die Referenzplatte 2 mit eindeutig definiertem Plattenkoordinatensystem R (Figur 5) dar, die während der Herstellung der Scanschablone 4 unlösbar und fest in diese integriert wird. Der Vorteil dabei ist, dass die Referenzplatte 2 bei der Befestigung gegenüber der Scanschablone 4 relativ frei positionierbar ist. Somit kann die Referenzplatte 2 abweichend von der Zahnebene und unter Berücksichtigung der Gegebenheiten im Mundraum angebracht werden. Eine Beeinflussung des Transformationsergebnisses z.B. durch Artefakte in der volumentomografischen Aufnahme kann dadurch minimiert oder eliminiert werden. Die Referenzplatte 2 wird aus einem nicht röntgenopaken festen Werkstoff gefertigt und dient somit gleichzeitig zur Stabilisierung der Scanschablone 4. Die Referenzplatte 2 weist ein eindeutiges, unsymmetrisches Schema von Bohrungen 8 zur Aufnahme von (mindestens 3) Referenzkugeln 10 aus röntgenopakem Material (Keramik, Si3N4) auf. Die Informationen zu Anzahl, Durchmesser und Lage der Kugeln 10 gegenüber dem Plattenkoordinatensystem R (Figur 5) sind bekannt und werden in einem Datenfile gespeichert.

Durch intelligente Kombination geeigneter Algorithmen werden die Positionen der Mittelpunkte der Kugeln 10 in der Referenzplatte 2 in Bezug auf das Referenzplattenkoordinatensystem R das ja auch das Koordinatensystem für den erzeugten Bildstapel 54 gemäß Figur 5 definiert, bestimmt. Dafür erfolgt eine erste Detektion der Kugelregionen innerhalb der einzelnen 2D-Schichtbilder durch die Bestimmung der Schnittkreise im schwellwertbasierten Kantenbild. Anschließend werden im dreidimensionalen Umfeld der gefundenen Regionen voxelbasiert die Kugelgeometrie und deren Mittelpunkt gesucht.

Aus dem Datenfile sind die Positionen der Kugeln 10 in der Referenzplatte 2 gegenüber dem Referenzplattenkoordinatensystem R (Figur 5) bekannt. Die Zuordnung der gefundenen Kugelmittelpunkte zu den bekannten Referenzkugelpositionen erfolgt aufgrund des oben genannten eindeutigen, unsymmetrischen Schemas der Bohrungen 8 in der Referenzplatte 2. Über die Korrespondenz der Mittelpunkte lässt sich eine Gesamttransformationsmatrix berechnen.

Wie bereits weiter oben angesprochen und insbesondere in Figur 3b erkennbar, zeichnet sich die Referenzplatte 2 weiterhin durch eine asymmetrische Anordnung der Montagebohrungen 12 aus, welche eine formschlüssige, wiederholbare Einspannung in einer speziellen dafür angepassten Aufnahmevorrichtung ermöglicht. Ein versehentliches Verdrehen der Platte 2 beim Einlegen ist ausgeschlossen. Die Aufnahmevorrichtung lässt sich einmalig durch Einmessen anhand bekannter technologischer Basen (z.B. Ebenen) in Bezug zur Plattenaufnahme innerhalb einer Werkzeugmaschine positionieren (Fertigungskoordinatensystem).

Die Anwendung im Gesamtkontext gestaltet sich wie folgt:
1. Anfertigung einer patientenspezifischen Scanschablone 4 anhand eines Kieferabdrucks 6 und/oder digitalen Mundscans;
2. Einarbeitung der Referenzplatte 2 mit Referenzkugeln 10 in die Scanschablone 4 (Fig.3);
3. Volumentomografische Aufnahme des Patienten mit Scanschablone 4;
4. Planung der medizinischen Versorgung (z.B. Implantate) im 3D-Volumendatensatz mit Hilfe einer Software in Abhängigkeit vom Koordinatensystem des Bildstapels 54 (Fig. 5);
5. Kugelfindung über oben beschriebenen Algorithmus im Bildstapel 54 und Berechnung der Mittelpunkte der Kugeln 10 in der Referenzplatte 2;
6. Anpassung an das Referenzplattenkoordinatensystem R (Fig. 5) über oben beschriebenen Ablauf (Fig. 5);
7. Export der transformierten relevanten Geometrie in einem (neutralen) Datenformat als Datei;
8. Import der Datei im CAM-System, Ermittlung der Bearbeitungsreihenfolge und Generierung eines CNC-Programms;
9. Einspannen der Scanschablone 4 in die oben beschriebene Aufnahmevorrichtung der NC-Maschine und Einbringen der Funktionselemente, die im dargestellten Ausführungsbeispiel Bohrungen 42 (Fig. 6) bilden, laut CAM-Planung, wodurch die Scanschablone 4 somit zur Positionierschablone umgearbeitet wird;
10. Nutzung der erstellten Positionierschablone 4 mit integrierten Funktionselementen (Bohrungen 42) zur dentalen Versorgung des Patienten.

Ein Aspekt ist die automatische Transformation von Koordinaten geplanter Funktionselemente vom Planungs- in das Fertigungskoordinatensystem unter Nutzung der Referenzplatte 2 mit definiert angeordneten Referenzkugeln 10, einem Algorithmus zur automatischen Kugeldetektion und Berechnung der Transformation sowie einer eindeutigen Aufnahmevorrichtung für die NC-Fertigung. Damit wird eine durchgängig digitale, dentale Versorgungsplanung und die Unterstützung der Versorgung durch eine CNC-gefertigte Positionierschablone mit integrierten Funktionselementen ermöglicht.

Als Vorteile ergeben sich:
- Automatische Transformation der Koordinaten ohne Zutun des Anwenders;
- Nutzung standardisierter Austauschformate zur Überführung der Daten von der Planung zur Fertigung;
- Kombination von Planung und Fertigung zu einem gesamteinheitlichen digitalen Workflow (durchgängige Technologie);
- Die Nutzung jeder mehrachsigen NC- oder CNC-Fräsmaschine zur Fertigung ist möglich aufgrund der Anwendung von CAM-Technologien und der Bereitstellung einer eindeutigen Aufnahmevorrichtung (kein Verdrehen möglich durch asymmetrische Stiftaufnahme);
- Freie Positioniermöglichkeit der Referenzplatte 2 gegenüber der Zahnebene;
- Zusätzliche Stabilisierung der Scan- bzw. Positionierschablone 4;
- Die Kugeln 10 werden in die Referenzplatte 2 eingepresst und können nach Sterilisierung erneut verwendet werden;
- Einfacher Aufbau der Platte 2 erlaubt kostengünstige Fertigungsmöglichkeit (generativ oder durch Fräsen);
- Der Einsatz standardisierter Kugeln 10, die nach vorgegebenen Toleranzklassen gefertigt wurden, ermöglicht gleichbleibende Qualität und Genauigkeit bei geringen Kosten.

Als Besonderheiten der dabei verwendeten Scan-Schablone 4 mit der Referenzplatte 2 ist folgendes festzuhalten:
- Referenzplatte 2 mit eindeutig definiertem Plattenkoordinatensystem R;
- Referenzplatte 2 weist ein eindeutiges, unsymmetrisches Schema von Bohrungen 8 zur Aufnahme von (bevorzugt mindestens 3) Referenzkugeln 10 aus röntgenopakem Material (bevorzugt Keramik, Si3N4) auf;
- Referenzplatte 2 zeichnet sich durch eine asymmetrische Anordnung der Montagebohrungen 12 aus, welche eine formschlüssige, wiederholbare Einspannung in einer speziellen dafür angepassten Aufnahmevorrichtung ermöglicht;
- Referenzplatte 2 wird aus einem nicht röntgenopaken festen Werkstoff gefertigt;
- Freie Positioniermöglichkeit der Referenzplatte 2 gegenüber der Zahnebene;
- Zusätzliche Stabilisierung der Scan- bzw. Positionierschablone 4;
- Die Kugeln 10 werden in die Referenzplatte 2 eingepresst und können nach Sterilisierung erneut verwendet werden;
- Einfacher Aufbau der Platte 2 erlaubt kostengünstige Fertigungsmöglichkeit (bevorzugt generativ oder durch Fräsen);
- Der Einsatz standardisierter Kugeln 10, die nach vorgegebenen Toleranzklassen gefertigt wurden, ermöglicht gleichbleibende Qualität und Genauigkeit bei geringen Kosten.

Durch intelligente Kombination geeigneter Algorithmen werden die Positionen der Kugelmittelpunkte gegenüber dem Referenzplattenkoordinatensystem R gemäß Fig. 5 bestimmt, an das das Koordinatensystem des Bildstapels 54 entsprechend angepasst ist. Dafür erfolgt eine erste Detektion der Kugelregionen innerhalb der gemeinsam den Bildstapel 54 bildenden einzelnen 2D-Schichtbilder (Fig. 5) durch die Bestimmung der Schnittkreise im schwellwertbasierten Kantenbild. Anschließend werden im dreidimensionalen Umfeld der gefundenen Regionen voxelbasiert (d.h. auf den Bilddaten, nicht auf Polygondaten wie z.B. STL) die Kugelgeometrie und deren Mittelpunkt gesucht. Es sind die Kugelpositionen gegenüber dem Referenzplattenkoordinatensystem R (Figur 5) bekannt. Die Zuordnung der gefundenen Kugelmittelpunkte zu den bekannten Referenzkugelpositionen erfolgt aufgrund des eindeutigen, unsymmetrischen Schemas der Bohrungen 8 der Referenzplatte 2. Über die Korrespondenz der Mittelpunkte der Kugeln 10 in der Referenzplatte 2 lässt sich eine Gesamttransformationsmatrix berechnen.

## Patentansprüche

1. Vorrichtung zur Verwendung in einem Verfahren zum Herstellen einer Zahnimplantatstruktur, mit
einer Abtasteinrichtung (20), die vorgesehen ist, den Kiefer eines Patienten abzutasten und daraus entsprechende Bilddaten zu erzeugen,
einer ersten Verarbeitungseinrichtung (22), die ausgebildet ist, in den Bilddaten aufgrund einer Festlegung einer Position, einer Orientierung und eines Raumes (50) für die Anordnung eines Implantates Implantatdaten zu erzeugen, die Parameter für die Anordnung des Implantats wie insbesondere die Abmessungen des hierfür festgelegten Raumes (50) angeben,
einer zweiten Verarbeitungseinrichtung (26), die ausgebildet ist, die Implantatdaten mit abgespeicherten Daten einer Vielzahl von unterschiedlichen Implantatausführungen zu vergleichen und als Ergebnis dieses Vergleiches mindestens eine passende Implantatausführung aus der Vielzahl der unterschiedlichen Implantatausführungen anzugeben, und
einer Transformationseinrichtung (34), die ausgebildet ist, die Implantatdaten auf der Grundlage eines Referenzkoordinatensystems (R) in Maschinendaten für eine im Zusammenhang mit dem Einsetzen des Implantates in den Kiefer zu verwendende Bearbeitungsmaschine (38) zu transformieren.

2. Vorrichtung nach Anspruch 1, bei welcher die erste Verarbeitungseinrichtung (22) ausgebildet ist, die Implantatdaten unter Berücksichtigung eines vorbestimmten Mindestabstandes zu einem benachbarten Hindernis wie insbesondere einem benachbarten Zahn, Implantat und/oder Nerv und/oder zu den Außenseiten des Kiefers zu erzeugen, so dass sie für die Anordnung eines Implantats nur einen festgelegten Raum (50) definieren, der in einem Abstand zu einem benachbarten Hindernis bzw. den Außenseiten des Kiefers liegt, welcher mindestens dem vorbestimmten Mindestabstand entspricht.

3. Vorrichtung nach mindestens einem der vorangegangen Ansprüche, mit einer dritten Verarbeitungseinrichtung (32), die ausgebildet ist, die Implantatdaten unter Verwendung der Daten der von der zweiten Verarbeitungseinrichtung (26) angegebenen passenden Implantatausführung entsprechend anzupassen.

4. Vorrichtung nach mindestens einem der vorangegangenen Ansprüche, bei welcher die zweite Verarbeitungseinrichtung (26) ausgebildet ist, als Ergebnis des Vergleiches aus der Vielzahl der unterschiedlichen Implantatausführungen eine Gruppe von passenden Implantatausführun-gen zur Auswahl durch den Anwender anzugeben.

5. Vorrichtung zur Verwendung in einem Verfahren zum Herstellen einer Zahnimplantatstruktur nach mindestens einem der vorangegangenen Ansprüche, mit
einem Referenzabschnitt (2), an oder in dem Referenzelemente (10) zur Definition eines Referenzkoordinatensystems (R) anzuordnen sind,
einem Schablonenabschnitt (4), der zur Aufnahme des Referenzabschnittes (2) in einer definierten Anordnung, zur lösbaren Befestigung am Kiefer eines Patienten und zur Aufnahme oder Ausbildung mindestens eines Zahnersatzteiles vorgesehen ist, und
Haltemitteln (12), die in einer definierten Position in Bezug auf die Referenzelemente (10) angeordnet und für ein lösbares Einspannen der Anordnung aus Schablonenabschnitt (4) und Referenzabschnitt (2) in einer Bearbeitungsmaschine (38), insbesondere einer CNC-Fräsmaschine, zum Einarbeiten mindestens eines Funktionselementes (42), bevorzugt einer Bohrung, zumindest in den Schablonenabschnitt (4) ausgebildet sind.

6. Vorrichtung nach Anspruch 5, bei welcher die Haltemittel (12) am oder im Referenzabschnitt (2) vorgesehen sind und/oder Aussparungen und/oder Löcher aufweisen.

7. Vorrichtung nach Anspruch 5 oder 6, bei welcher für die Referenzelemente (10) eine asymmetrische Anordnung vorgesehen ist.

8. Vorrichtung nach mindestens einem der Ansprüche 5 bis 7, bei welcher im Referenzabschnitt (2) im Querschnitt kreisförmige Löcher oder Aussparungen (8) zur, bevorzugt lösbar, arretierenden Aufnahme von als Kugeln ausgebildeten Referenzelementen (10) vorgesehen sind.

9. Vorrichtung nach mindestens einem der Ansprüche 5 bis 7, bei welcher der Referenzabschnitt (2) im Wesentlichen als Platte ausgebildet ist.

10. Vorrichtung nach mindestens einem der Ansprüche 5 bis 8, bei welcher der Referenzabschnitt (2), der Schablonenabschnitt (4) und die Haltemittel (12) miteinander einstückig ausgebildet sind.

11. Vorrichtung nach mindestens einem der Ansprüche 5 bis 8, bei welcher der Referenzabschnitt (2) lösbar am Schablonenabschnitt (4) angeordnet ist.

12. Vorrichtung nach mindestens einem der Ansprüche 5 bis 11, mit Befestigungsmitteln, insbesondere Klebemitteln, zur Befestigung mindestens eines Zahnersatzteils (4a) am Schablonenabschnitt (4).

13. Vorrichtung nach mindestens einem der Ansprüche 5 bis 12, bei welcher der Referenzabschnitt (2), der Schablonenabschnitt (4) und die Haltemittel (12) im Wesentlichen nicht abbildbares Material und die Referenzelemente (10) und das mindestens eine Zahnersatzteil (4a) im Wesentlichen abbildbares Material aufweisen.

14. Vorrichtung nach mindestens einem der Ansprüche 5 bis 13, bei welcher der Schablonenabschnitt (4) im Wesentlichen eine entsprechend dem Kiefer (6) gekrümmte Formgebung aufweist.

15. Vorrichtung nach mindestens einem der Ansprüche 5 bis 14, bei welcher der Schablonenabschnitt (4) zur Einarbeitung mindestens eines als Durchgangsbohrung ausgebildeten Funktionselementes (42) für die Aufnahme einer Bohrerführungshülse (44) vorgesehen ist.

16. Vorrichtung nach mindestens einem der Ansprüche 5 bis 15, **dadurch gekennzeichnet, dass** der Schablonenabschnitt (4) Befestigungsmittel, insbesondere Ankerpins, zur lösbaren Befestigung in einer definierten Anordnung am Kiefer des Patienten aufweist.

17. Verfahren zur Herstellung einer Zahnimplantatstruktur, insbesondere unter Verwendung einer Vorrichtung nach mindestens einem der Ansprüche 1 bis 4, mit den Schritten,
A) mit einer Abtasteinrichtung (20) Bilddaten vom Kiefer eines Patienten zu erzeugen,
B) in den Bilddaten aufgrund einer Festlegung der Position, der Orientierung und des Raumes (50) für die Aufnahme eines Implantates Implantatdaten zu erzeugen, die Parameter für die Anordnung des Implantats wie insbesondere die Abmessungen des hierfür festgelegten Raumes (50) angeben,
C) die Implantatdaten mit abgespeicherten Daten einer Vielzahl von unterschiedlichen Implantatausführungen zu vergleichen und als Ergebnis dieses Vergleiches mindestens eine passende Implantatausführung aus der Vielzahl der unterschiedlichen Implantatausführungen anzugeben, und
D) die Implantatdaten auf der Gundlage eines Referenzkoordinatensystems (R) in Maschinendaten für eine im Zusammenhang mit dem Einsetzen des Implantates in den Kiefer zu verwendende Bearbeitungsmaschine (38) zu transformieren.

18. Verfahren nach Anspruch 17, bei welchem im Schritt B die Implantatdaten unter Berücksichtigung eines vorbestimmten Mindestabstandes zu einem benachbarten Hindernis wie insbesondere einem benachbarten Zahn, Implantat und/oder Nerv und/oder zu den Außenseiten des Kiefers erzeugt werden, so dass sie für die Anordnung eines Implantats nur einen solchen Raum (50) definieren, der in einem Abstand zu einem benachbarten Hindernis bzw. den Außenseiten des Kiefers liegt, welcher mindestens dem vorbestimmten Mindestabstand entspricht.

19. Verfahren nach Anspruch 17 oder 18, bei welchem im Schritt C als Ergebnis des Vergleiches aus der Vielzahl der unterschiedlichen Implantatausführungen eine Gruppe von passenden Implantatausführungen zur Auswahl durch den Anwender angegeben wird.

20. Verfahren nach mindestens einem der Ansprüche 17 bis 19, bei welchem zwischen dem Schritt C und dem Schritt D die Implantatdaten unter Berücksichtigung der Daten der passenden Implantatausführung entsprechend angepasst werden.

21. Verfahren zur Herstellung einer Zahnimplantatstruktur unter Verwendung einer Vorrichtung nach mindestens einem der Ansprüche 5 bis 16, mit den Schritten,
den Schablonenabschnitt (4) mit mindestens einem Zahnersatzteil zu versehen,
von einem Kiefer des Patienten mit dem daran befestigten Schablonenabschnitt (4) Bilddaten zu erzeugen,
auf der Grundlage der in den Bilddaten enthaltenen Daten für die Referenzelemente (10) ein Referenzkoordinatensystem (R) zu definieren,
in den Bilddaten aufgrund einer Festlegung einer Position, einer Orientierung und eines Raumes (56) für die Anordnung eines Implantates entsprechende Implantatdaten zu erzeugen,
die Implantatdaten auf der Grundlage des Referenzkoordinatensystems (R) in Maschinendaten zu transformieren,
die Anordnung aus Schablonenabschnitt (2) und Referenzabschnitt (4) mit den Haltemitteln (12) in eine Bearbeitungsmaschine (38) einzuspannen und mit Hilfe der Bearbeitungsmaschine (38) unter Verwendung der Maschinendaten mindestens ein Funktionselement (42), bevorzugt eine Bohrung, zumindest in den Schablonenabschnitt (4) der Vorrichtung einzuarbeiten.

22. Verfahren nach mindestens einem der Ansprüche 17 bis 20 unter Verwendung einer Vorrichtung nach mindestens einem der Ansprüche 8 bis 20, bei welchem
vor dem Schritt A der Schablonenabschnitt (4) mit mindestens einem Zahnersatzteil (4a) versehen wird,
zwischen dem Schritt A und dem Schritt B auf der Grundlage der in den Bilddaten enthaltenen Daten für die Referenzelemente (10) ein Referenzkoordinatensystem (R) definiert wird, und
nach dem Schritt D die Anordnung aus Schablonenabschnitt (4) und Referenzabschnitt (2) mit den Haltemitteln (12) in eine Bearbeitungsmaschine (38) eingespannt und mit Hilfe der Bearbeitungsmaschine (38) unter Verwendung der Maschinendaten mindestens ein Funktionselement (42), bevorzugt eine Bohrung, zumindest in den Schablonenabschnitt (4) der Vorrichtung eingearbeitet wird.

23. Verfahren nach Anspruch 21 oder 22, mit dem weiteren Schritt, den Referenzabschnitt (2) vom Schablonenabschnitt (4) zu trennen.
